(19) Europäisches Patentamt
European Patent Office
Office européen des brevets

(11) Publication number: 0 020 107
B1

(12) EUROPEAN PATENT SPECIFICATION

(45) Date of publication of patent specification: 11.08.82

(51) Int. Cl.[3]: C 07 D 223/16,
A 61 K 31/55

(21) Application number: 80301716.9

(22) Date of filing: 23.05.80

(54) 3-Allyl-7,8-dihydroxy-6-halo-1-(4-hydroxyphenyl)-2,3,4,5-tetrahydro-1H-3-benzazepine derivatives, process for preparing them and compositions containing them.

(30) Priority: 25.05.79 US 42680

(43) Date of publication of application:
10.12.80 Bulletin 80/25

(45) Publication of the grant of the patent:
11.08.82 Bulletin 82/32

(84) Designated Contracting States:
AT BE CH DE FR GB IT LI LU NL SE

(56) References cited:
EP - A - 0 004 794
BE - A - 860 774
FR - A - 2 384 759
US - A - 4 104 379
US - A - 4 108 989

(73) Proprietor: SMITHKLINE CORPORATION
1500 Spring Garden Street P.O. Box 7929
Philadelphia Pennsylvania 19101 (US)

(72) Inventor: Weinstock, Joseph
1234 Pothouse Road
Phoenixville, Pennsylvania 19405 (US)

(74) Representative: Claisse, John Anthony, Dr. et al,
P.O. Box 39 Welwyn Garden City
Hertfordshire AL7 1EY (GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 3-Allyl-7,8-dihydroxy-6-halo-1-(4-hydroxyphenyl)-2,3,4,5-tetrahydro-1H-3-benzazepine derivatives, process for preparing them and compositions containing them

This invention concerns specific 7,8-dihydroxy-6-halo-1-phenyl-2,3,4,5-tetrahydro-1H-3-benzazepine derivatives which are renal vasodilators showing a novel bradycardic effect. As such they are of particular interest for treating angina pectoris.

Swiss patent Specification 555,831 and Belgian patent Specification 860,774 disclose generic groups of compounds related to those of the present invention but they do not disclose the specific substitution or their unique biological activity.

According to the present invention there are provided compounds represented by the formula:

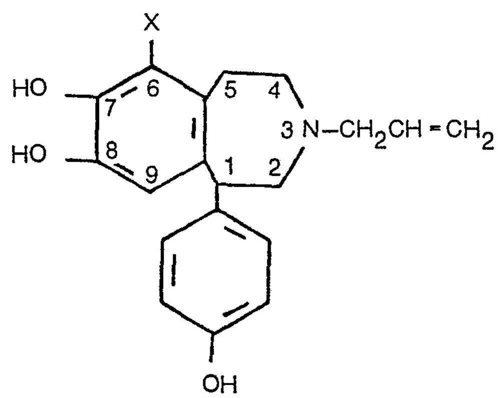

I

in which X is halo, that is fluoro, chloro, bromo or iodo, and pharmaceutically acceptable acid addition salts thereof.

The pharmaceutically acceptable acid addition salts in general have the utility of the free bases of Formula I, and can be prepared by methods well known to the art with both inorganic and organic acids, for example with maleic, fumaric, benzoic, ascorbic, pamoic, succinic, bismethylenesalicylic, methanesulfonic ethanedisulfonic, acetic, oxalic, propionic, tartaric, salicylic, citric, gluconic, aspartic, stearic, palmitic, itaconic, glycolic, p-aminobenzoic, glutamic, benzenesulfonic, hydrochloric, hydrobromic, sulfuric, cyclohexylsulfamic, phosphoric or nitric acids. The free base is conveniently reacted with an equivalent or an excess of the chosen acid in an organic solvent. The salt can then be isolated by filtration or evaporation of the solvent. The hydrohalic and especially methane-sulfonic acid salts are of particular utility because of good solubility and oral activity.

Also included in this invention are O-lower alkanoyl esters of the compounds of Formula I and their pharmaceutically acceptable acid addition salts. The alkanoyl groups each having from 2—8 carbon atoms, for example acetyl, isobutyryl, propionyl, isovaleryl, n-heptanoyl and other alkanoyl groups. The triester derivatives are exemplified herein but one or more hydroxy groups in formula I can be esterified stepwise, using synthetic methods known to the art. The ester derivatives can be prepared by reacting an appropriate trihydroxy compound of Formula I with either a stoichiometric amount or an excess of an alkanoyl bromide or an appropriate acid anhydride in the presence of an organic base, optionally in an organic solvent. Acetonitrile is a preferred solvent. Reference hereinafter to compounds of Formula I is intended to include not only the free bases but such O-lower alkanoyl esters and/or salts thereof.

The compounds of Formula I can exist as diastereoisomers which can be resolved into *d, 1* optical isomers. Resolution of the optical isomers can be conveniently accomplished by fractional crystallization of their salts with optically active acids from appropriate solvents. Unless otherwise specified herein or in the claims, it is intended to include all isomers, whether separated or mixtures thereof. Where isomers are separated, the desired pharmacological activity will usually predominate in one of the isomers, however a racemic mixture of isomer will usually be used.

The compounds of Formula I can be prepared by reaction of a compound of the formula:

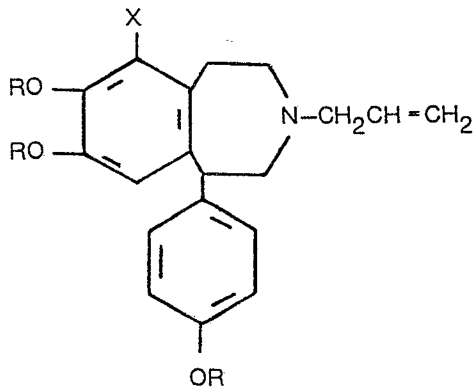

II

in which X is halo, and each R, which are the same or different, is a lower alkyl group, especially containing from 1 to 6 carbon atoms, for example a methyl group; a benzyl group or, when the two substituents R on the benz ring are taken together, a methylene or ethylene group; with an ether cleaving agent, for example boron tribromide or trichloride, preferably in a halogenated organic solvent, for example methylene chloride, carbon tetrachloride or chloroform; pyridine hydrochloride; aqueous hydrogen bromide; aluminum chloride or bromide in a suitable solvent, for example benzene or carbon disulfide; hydriodic acid; hydrogen fluoride-antimony pentafluoride; or trifluoromethylsulfonic acid in thioanisole to give the desired compound of Formula I. Surprisingly the 3- or N-allyl group is relatively unreactive however it is preferred to keep the reaction conditions as mild as possible to minimize reaction at the double bond of the allyl group.

The N-allyl intermediates of formula II can be prepared by N-allylation of a compound of formula:

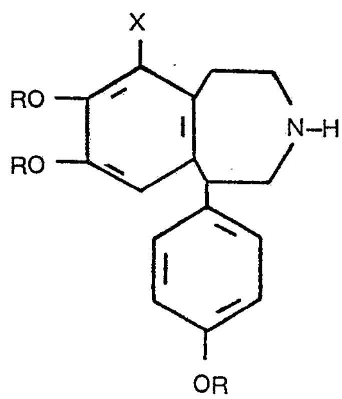

III

in which X and R are as defined above, using allyl bromide, chloride or iodide, preferably in an inert organic solvent and especially in acetonitrile, in the presence of a tertiary organic base, for example pyridine, triethylamine or dimethylaniline

Biological Spectrum of the Compounds

The compounds of Formula I have been unexpectedly found to have a unique hemodynamic profile that is characterized by reduced arterial blood pressure, reduced cardiac rate and reduced myocardial work with an augmented heart stroke volume and increased renal perfusion.

The compounds therefore are especially useful as anti-anginal agents.

The spectrum of activity of these new compounds is demonstrated by three pharmacological procedures: (1) The normotensive anesthetized dog test protocol where mean arterial blood pressure (MAP), renal blood flow (RBF), renal vascular resistance (RVR) and heart rate (HR) are monitored after infusion in μg/kg/min doses using the known pharmacodynamic procedure for assaying peripheral dopaminergic effects especially on the kidney, (2) the turning rat anti-Parkinsonism test for central dopaminergic effects, and (3) the spontaneously hypertensive rat procedure.

The following data illustrates the desirable spectrum of activity of the compounds of this invention compared with that of related compounds whose structures are considered closer to those claimed than are the structures specifically disclosed in the prior art.

Test 1 — normotensive anesthetized dog

(A) 3-allyl-6-chloro-7,8-dihydroxy-1(4-hydroxyphenyl)-2,3,4,5-tetrahydro-1H-3-benzazepine hydrobromide

| μg/kg/min | MAP | RBF | RVR | HR |
|---|---|---|---|---|
| 0.3 | − 3.5 | + 8.7* | −11.4* | 0 |
| 3 | −14.4* | +18.1* | −27.8* | +0.5 |
| 30 | −16.9* | +20.0* | −30.7* | −8.7* |

(B) 3-allyl-6-chloro-7,8-dihydroxy-1-phenyl-2,3,4,5-tetrahydro-1H-3-benzazepine hydrobromide hydrate

| μg/kg/min | MAP | RBF | RVR | HR |
|---|---|---|---|---|
| 0.3 | − 1.8 | +13.0* | −13.1 | + 6.8* |
| 3 | − 8.5* | +32.5* | −30.7* | + 7.4* |
| 30 | −22.3* | + 6.6* | −22.3* | − 1.0 |
| 300 | + 3.4 | −12.5* | +18.3* | −13.5* |

(C) 6-chloro-7,8-dihydroxy-3-methyl-1(4-hydroxyphenyl)-2,3,4,5-tetrahydro-1H-3-benzazepine hydrobromide

| μg/kg/min | MAP | RBF | RVR | HR |
|---|---|---|---|---|
| 3 | − 3.3 | 0 | −3.0 | 0 |
| 30 | + 4.5 | + 7.1 | −2.4 | 0 |
| 300 | +11.1* | +17.4* | −5.1 | 0 |

(D) 6-chloro-7,8-dihydroxy-1-(4-hydroxyphenyl)-2,3,4,5-tetrahydro-1H-3-benzazepine

| μg/kg/min | MAP | RBF | RVR | HR |
|---|---|---|---|---|
| 3 | − 6.7 | +29.4 | −26.2* | + 6.7* |
| 30 | − 6.8 | +18.7* | −21.1* | +14.2* |
| 300 | −18.3* | − 9.8* | −10.4* | +18.2* |

The starred numbers are considered significant

These data demonstrate that at 30 micrograms the above 6-chloro compound of this invention (Compound A) demonstrated renal dopaminergic effects with increased kidney blood flow, as well as lower arterial blood pressure and bradycardia. Compound B whose structure lacks the 4′-phenolic hydroxy group of Compound A demonstrates no bradycardia at 30 micrograms and at 300 micrograms demonstrates bradycardia but no renal dopaminergic activity. Compound C which has a 3-methyl group in place of the 3-allyl group demonstrates no renal dopaminergic or bradycardic activity. Compound D which lacks an N-allyl group demonstrates *increased* heart rate at all doses. The latter is the expected reaction to general peripheral vasodilation.

Test 2 — Rat rotation (i.p.)

| | | | | |
|---|---|---|---|---|
| (A) | 10 mg/kg | 647 ± 84 | (B) $RD_{500}$ | 0.03 mg/kg |
| | 10 | 631 ± 152 | | |
| | 2.0 | 297 ± 64 | | |
| | Significant but weak activity. | | | |
| (C) | 10 mg/kg | 762 ± 253 | (D) 10 mg/kg | 14 ± 7 |
| | weak activity | | inactive | |

Test 3 — Spontaneously hypertensive rat
Protocol:

Adult male spontaneously hypertensive rats, weighing about 350—400 grams, are anesthetized with sodium pentobarbital (65 mg/kg, i.p.). The trachea are cannulated and the rats are allowed to respire spontaneously. Pulsatile arterial blood pressure is measured from a cannulated carotid artery using a transducer. Mean arterial blood pressure is calculated as diastolic blood pressure plus 1/3 pulse pressure. Cardiac rate is monitored by a cardiotachometer triggered by the systotic blood pressure pulse. Drug solutions are administered through a cannulated tail vein. Following surgery, approximately 10 minutes are allowed to elapse for equilibration. The test compounds are administered in 0.9% saline. The results are recorded as mcg/kg of free base. Control tracings are taken and readings are taken at 5—10 minute intervals with increasing doses from 1—1000 mcg/kg.

Compound A of this invention decreased arterial blood pressure and cardiac rate in a dose-related manner over 1—1000 mcg/kg (i.v.). At the same dose range dopamine produced prominent tachycardia and arterial hypertension. The bradycardia and hypotension demonstrated by Compound A were antagonized by a ganglionic blocker (hexamethonium) and a dopamine antagonist (metoclopromide). Compound A at an intraduodenal dose of 10 mg/kg in the anesthetized dog produced bradycardia and arterial hypotension of 30—45 minutes duration. At 5 mg/kg orally in the normal dog no side effects were observed.

Compound B, the deshydroxy congener, also produced bradycardia and hypotension in the SH rat but did not have a specificity of action since it was a very powerful central dopaminergic agent.

Compound C at from 1—1000 mg/kg (i.v.) exhibited only trivial blood pressure effects and decreased cardiac rate only at the highest dose tested (1000 mcg/kg).

In addition to the tests outlined above, detailed study of Compound A in the anesthetized dog demonstrated a reduced peripheral resistance in the systemic vasculature, increased stroke volume of the heart despite slowing and possibly a decreased adrenergic tone to the heart and vasculature.

The invention therefore provides pharmaceutical compositions comprising a compound of the present invention and a pharmaceutical carrier. The compositions can be used to obtain a unique antihypertensive activity and especially activity against angina pectoris. In general, conventional dosage unit forms will be used and these can be prepared according to accepted procedures. The compound of the invention will in general be present in each dosage unit in a nontoxic amount sufficient to produce the desired pharmacodynamic activity in a subject, animal or human. The dosage units will usually contain the active ingredient in an active but nontoxic amount selected from about 25 mg to about 500 mg, preferably about 15—75 mg of active ingredient per dosage unit, but this quantity will depend on the potency of the compound, the specific biological activity desired, the route of administration (oral or parenteral) and the condition of the patient. Compound (A) of this invention, especially as its methyl sulfonate salt, has been found to have good absorbability from the gastrointestinal tract, so oral dosage forms are especially preferred preferably selected from the dosage unit ranges given above. Intravenous or subcutaneous doses would be lower.

The pharmaceutical compositions can be made following the conventional techniques of the pharmaceutical chemist involving mixing, granulating and compressing when necessary, or variously mixing and dissolving the ingredients as appropriate to give the desired end product.

The following Examples illustrate the preparation and use of compounds of this invention. The temperatures are in degrees Centigrade. Other variations of these Examples will be obvious to those skilled in the art.

## Example 1

6-Chloro-7,8-dimethoxy-1-(4-methoxyphenyl)-2,3,4,5-tetrahydro-1H-3-benzazepine (m.p. 140—142.5°, 3.47 g, 0.01 m) in 50 ml of acetonitrile was mixed with 2.8 ml (0.02 m) of triethylamine and 1.4 ml (0.011 m) of allyl bromide. The mixture was heated at 85—95° for 2-1/2 hours. The reaction mixture was evaporated. The residue was suspended in water and extracted twice with ethyl acetate. The organic extracts were washed with water, brine and evaporated to give 2.6 g (67.2%) of a

yellow oil, 3-allyl-6-chloro-7,8-dimethoxy-1-(4-methoxyphenyl)-2,3,4,5-tetrahydro-1H-3-benzazepine.

This material (2.6 g, 0.0067 m) was dissolved in 55 ml of methylene chloride and cooled to —15° at which time 6.0 ml (0.064 m) of boron tribromide in 40 ml of methylene chloride was added slowly over 1/2 hour. The reaction mixture was stirred at room temperature for 3 hours, cooled and treated with an excess of methanol slowly and with cooling. The methanol was evaporated to give a foam. This was dissolved in a minimum amount of methanol and cooled. Some ethyl acetate was added to induce separation of 1.85 g (65%) of 3-allyl-6-chloro-7,8-dihydroxy-1-(4-hydroxyphenyl)-2,3,4,5-tetrahydro-1H-3-benzazepine hydrobromide, m.p. 195—199° (dec.).

An aliquot of the hydrobromide (1 g) in aqueous methanol is neutralized using sodium carbonate solution. The base (500 mg) is reacted with a slight excess of methane sulfonic acid in methanol to give the methylsulfonate salt (550 mg).

Using 6-fluoro-7,8-dimethoxy-1-(4-methoxyphenyl-2,3,4,5-tetrahydro-1H-3-benzazepine for N-allylation and subsequent demethylation with boron tribromide as above gives 3-allyl-6-fluoro-7,8-dihydroxy-1-(4-hydroxyphenyl)-2,3,4,5-tetrahydro-1H-3-benzazepine hydrobromide. Substituting 6-bromo-7,8-dimethoxy-1-(4-methoxyphenyl)-2,3,4,5-tetrahydro-1H-3-benzazepine in the above procedures using N-allylation and dealkylation with boron tribromide gives 3-allyl-6-bromo-7,8-dihydroxy-1-(4-hydroxyphenyl)-2,3,4,5-tetrahydro-1H-3-benzazepine hydrobromide.

Example 2

The 6-bromo-trimethoxybenzazepine starting material from Example 1 (7.8 g, 0.018 m) in 300 ml of methylene chloride and excess trifluoroacetic acid anhydride was stirred for 2 hours. The solvent was evaporated, and toluene was added. The mixture was again stripped to give the 3-amide. A mixture of 25 ml of butyl lithium (2.6 M, 0.65 m) in 200 ml of ether was cooled to —78° and the 3-amide (8.7 g) added in 60 ml of toluene. After stirring for 15 minutes a mixture of 9.1 g of iodine (0.036 m) and 60 ml of ethyl ether was added. After stirring while warming up to room temperature, the mixture was reacted with 200 ml of 10% hydrochloric acid at 0°. The mixture was stirred. The solid collected was taken into methylene chloride/methanol, washed with brine, dried and evaporated to give 6-iodo-7,8-dimethoxy-1-(4-methoxyphenyl)-2,3,4,5-tetrahydro-1H-3-benzazepine hydrochloride, m.p. 237—239. N-Allylation using allyl iodide on 2 g of the trimethoxy compound followed by demethylation of 1.25 g of the N-allyl compound as in Example 1 gives 450 mg of 3-allyl-7,8-dihydroxy-6-iodo-1-(4-hydroxyphenyl)-2,3,4,5-tetrahydro-1H-3-benzazepine hydrobromide.

Example 3

3-Allyl-6-chloro-7,8-dihydroxy-1-(4-hydroxyphenyl-2,3,4,5-tetrahydro-1H-3-benzazepine hydrobromide (1.0 g) is slurried in 200 ml of trifluoroacetic acid then 1.29 ml of acetyl bromide is added. The mixture is heated at reflux for 2 hours then stirred for 2 hours. After evaporation to dryness the residue is taken up in benzene and concentrated to give 3-allyl-6-chloro-7,8-diacetoxy-1-(4-acetoxyphenyl-2,3,4,5-tetrahydro-1H-3-benzazepine hydrobromide.

Similarly the isobutyryloxy, propionyloxy, isovaleryloxy, n-butyryloxy, n-heptanoyloxy and other higher derivatives are prepared at the catecholic hydroxy groups or at all three hydroxy sites.

Example 4

| Ingredients | Mg. per Capsule |
|---|---|
| 3-Allyl-6-chloro-7,8-dihydroxy-1-(4-hydroxyphenyl)-2,3,4,5-tetrahydro-1H-3-benzazepine hydrobromide | 50 (free base) |
| Magnesium stearate | 2 |
| Lactose | 150 |

The above ingredients are thoroughly mixed and placed into hard gelatin capsules. Such capsules are administered orally to subjects in need of treatment from 1—5 times daily to induce a specific dopaminergic activity to treat the symptoms of angina pectoris.

**Claims for the Contracting States: BE, CH, LI, DE, FR, GB, IT, LU, NL, SE.**

1. A compound of the formula:

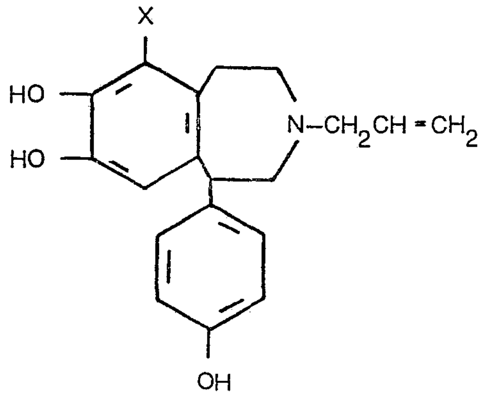

I

in which X is halo; together with a pharmaceutically acceptable acid addition salt or O-lower alkanoyl ester thereof, the alkanoyl groups each having 2.8 carbon atoms.

2. The compound of claim 1 being 3-allyl-6-chloro-7,8-dihydroxy-1-(4-hydroxyphenyl)-2,3,4,5-tetrahydro-1H-3-benzazepine.

3. The compound of claim 1 being 3-allyl-6-iodo-7,8-dihydroxy-1-(4-hydroxyphenyl)-2,3,4,5-tetrahydro-1H-3-benzazepine.

4. The compound of claim 2 as the methanesulfonate salt.

5. The compound of claim 2 as the hydrochloride or hydrobromide salt.

6. A process for preparing a compound according to any of the preceding claims which comprises reacting a compound of the formula:

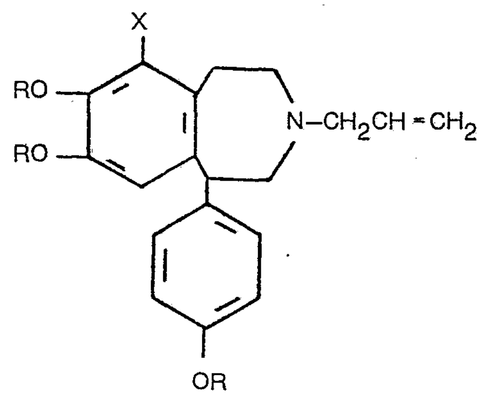

II

in which X is halo and each R, which are the same or different, is a lower alkyl group containing from 1 to 6 carbons, a benzyl group, or, when taken together at the 7,8-positions of the benz ring, methylene or ethylene, with an ether cleaving agent.

7. A process according to claim 6, in which the ether cleaving agent is boron tribromide in a halogenated organic solvent.

8. The pharmaceutical composition having antiangina pectoris activity comprising a compound of claims 1, 2, 3, 4 or 5 combined with a pharmaceutical carrier.

9. The pharmaceutical composition in dosage unit form having antianginal pectoris activity comprising a compound of any one of claims 1 to 5 in a non-toxic amount sufficient to produce antiangina pectoris activity in a human, combined with a pharmaceutical carrier.

10. The pharmaceutical composition of claim 9 comprising from 15 to 75 mg of a compound of any one of claims 1 to 5 per dosage unit.

**Claims for the Contracting State: AT.**

1. A process for preparing a compound of the formula:

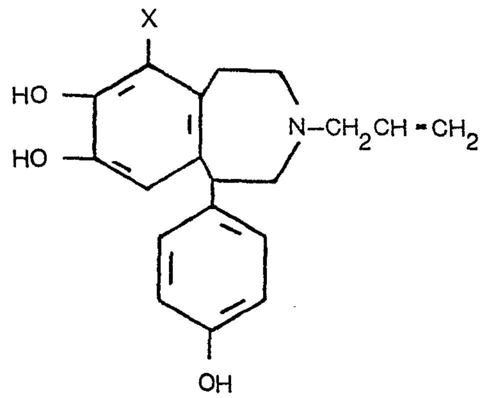

I

in which X is halo; together with a pharmaceutically acceptable acid addition salt of O-lower alkanoyl ester thereof, the alkanoyl groups each having 2—8 carbon atoms, which comprises reacting a compound of the formula:

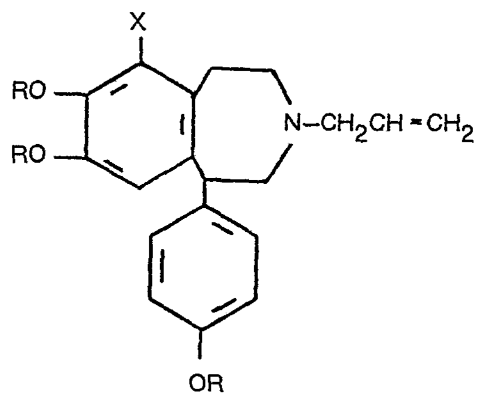

II

in which X is halo and each R, which are the same or different, is a lower alkyl group containing from 1 to 6 carbons, a benzyl group, or, when taken together at the 7,8-positions of the benz ring, methylene or ethylene, with an ether cleaving agent, and optionally converting the product into a pharmaceutically acceptable acid addition salt or O-lower alkanoyl ester.

2. A process according to Claim 1 in which X is chloro.

3. A process according to Claim 1 in which X is iodo.

4. A process according to any one of Claims 1 to 3 in which all three R groups are methyl.

5. A process according to any one of Claims 1 to 4 in which the ether cleaving agent is boron tribromide in a halogenated organic solvent.

**Patentansprüche für die Vertragsstaaten: BE, CH, LI, DE, FR, GB, IT, LU, NL, SE.**

1. Eine Verbindung der allgemeinen Formel I

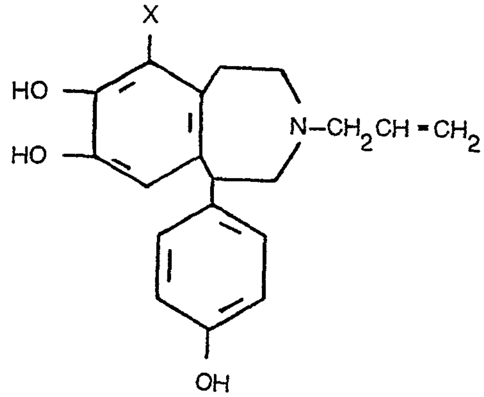

(I)

in der X ein Halogenatom bedeutet, zusammen mit ihrem pharmazeutisch verträglichen Säureadditionssalz oder O-Niederalkanoylester, wobei der Alkanoylrest jeweils 2 bis 8 Kohlenstoffatome aufweist.

2. Verbindung nach Anspruch 1, nämlich 3 - Allyl - 6 - chlor - 7,8 - dihydroxy - 1 - (4 - hydroxyphenyl) - 2,3,4,5 - tetrahydro - 1H - 3 - benzazepin.

3. Verbindung nach Anspruch 1, nämlich 3 - Allyl - 6 - jod - 7,8 - dihydroxy - 1 - (4 - hydroxyphenyl) - 2,3,4,5 - tetrahydro - 1H - 3 - benzazepin.

4. Verbindung nach Anspruch 2 als Methansulfonatsalz.

5. Verbindung nach Anspruch 2 als Chlorwasserstoff- oder Bromwasserstoffsalz.

6. Verfahren zur Herstellung einer Verbindung nach einem der vorstehenden Ansprüche, dadurch gekennzeichnet, daß man eine Verbindung der allgemeinen Formel II

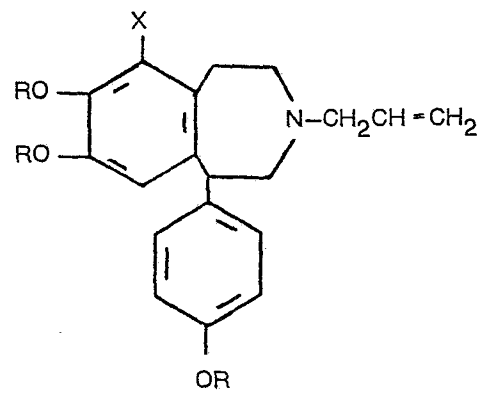

(II)

in der X ein Halogenatom bedeutet und die Reste R, die gleich oder verschieden sind, einen Niederalkylrest mit 1 bis 6 Kohlenstoffatomen, eine Benzylgruppe oder in der 7,8-Stellung zusammen eine Methylen- oder Äthylengruppe darstellen, mit einem Mittel an der Ätherbindung spaltet.

7. Verfahren nach Anspruch 6, dadurch gekennzeichnet, daß das Mittel zur Spaltung der Ätherbindung Bortribromid in einem halogenierten organischen Lösungsmittel ist.

8. Arzneimittel zur Bekämpfung von Angina pectoris, enthaltend eine Verbindung der Ansprüche 1, 2, 3, 4, oder 5 und einen pharmazeutischen Trägerstoff.

9. Arzneimittel in Verabreichungsform mit Wirksamkeit gegen Angina pectoris, enthaltend eine Verbindung nach den Ansprüchen 1 bis 5 in einer nichttoxischen Menge, die ausreicht, Wirkung gegen Angina pectoris beim Menschen zu entfalten, in Verbindung mit einem pharmazeutischen Trägerstoff.

10. Arzneimittel nach Anspruch 9, enthaltend 15 bis 75 mg einer Verbindung der Ansprüche 1 bis 5 als Dosiseinheit.

**Patentansprüche für den Vertragsstaat: AT**

1. Verfahren zur Herstellung einer Verbindung der allgemeinen Formel I

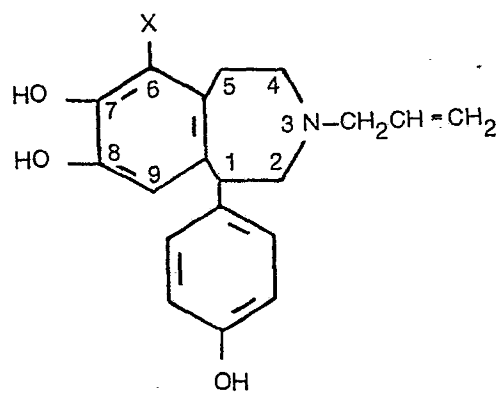

(I)

in der X ein Halogenatom darstellt, und ihre pharmazeutisch verträglichen Säureadditionssalze oder O-Niederalkanoylester, wobei die Alkanoylreste jeweils 2 bis 8 Kohlenstoffatome aufweisen, dadurch gekennzeichnet, daß man eine Verbindung der allgemeinen Formel II

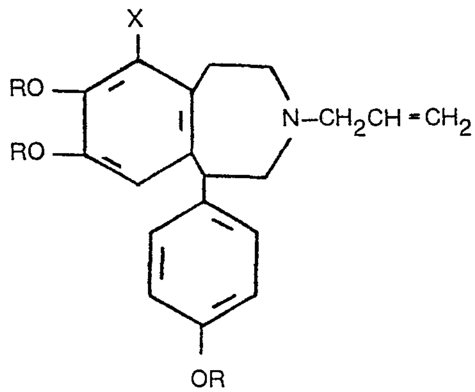

(II)

in der X ein Halogenatom und die Reste R, die gleich oder verschieden sind, einen Niederalkylrest mit 1 bis 6 Kohlenstoffatomen, eine Benzylgruppe oder in der 7,8-Stellung zusammen eine Methylen- oder Äthylengruppe darstellen, mit einem Mittel zur Spaltung der Ätherbindung behandelt und gegebenenfalls das erhaltene Produkt in das pharmazeutisch verträgliche Säureadditionssalz oder den O-Niederalkanoylester überführt.

2. Verfahren nach Anspruch 1, wobei X ein Chloratom ist.

3. Verfahren nach Anspruch 1, wobei X ein Jodatom ist.

4. Verfahren nach einem der Ansprüche 1 bis 3, wobei alle drei Reste R eine Methylgruppe bedeuten.

5. Verfahren nach einem der Ansprüche 1 bis 4, wobei das Mittel zur Spaltung der Ätherbindung Bortribromid in einem halogenierten organischen Lösungsmittel ist.

**Revendications pour les Etats contractants: BE, CH, LI, DE, FR, GB, IT, LU, NL, SE.**

1. Composé de formule

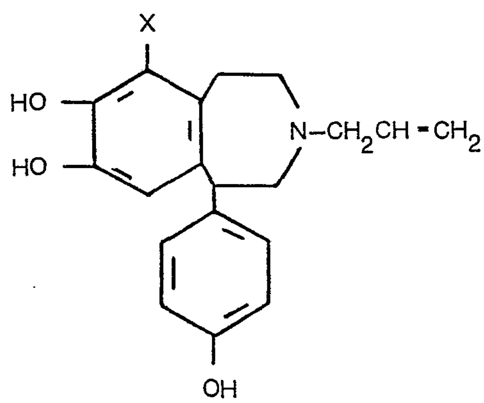

I

dans laquelle X représente un groupe halo; ainsi qu'un sel d'addition de ce composé avec des acides pharmaceutiquement acceptables ou son ester O-alcanoylé inférieur, chacun des groupes alcanoylés ayant de 2 à 8 atomes de carbone.

2. Composé suivant la revendication 1, qui est la 3 - allyl - 6 - chloro - 7,8 - dihydroxy - 1 - (4 - hydroxyphényl) - 2,3,4,5 - tétrahydro - 1H- 3 - benzazépine.

3. Composé suivant la revendication 1, qui est la 3 - allyl - 6 - iodo - 7,8 - dihydroxy - 1 - (4 - hydroxyphényl) - 2,3,4,5 - tétrahydro - 1H - 3 - benzazépine.

4. Composé suivant la revendication 2, sous la forme de méthanesulfonate.

5. Composé suivant la revendication 2, sous la forme de chlorhydrate ou de bromhydrate.

6. Procédé de préparation d'un composé suivant l'une quelconque des revendications précédentes qui consiste à faire réagir un composé de formule:

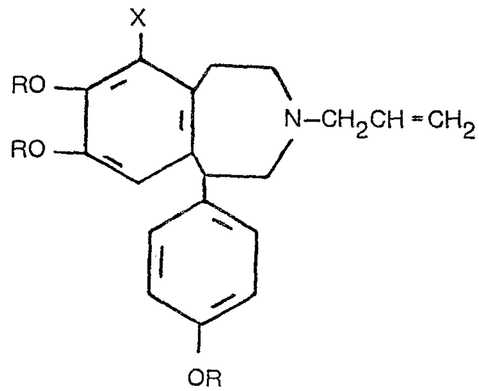

II

dans laquelle X représente un groupe halo et chacun des groupes R, qui sont identiques ou différents, représente un groupe alcoyle inférieur contenant de 1 à 6 atomes de carbon, un groupe benzyle, ou lorsqu'ils sont considérés conjointement aux positions 7,8 due cycle benzénique, représentent un groupe méthylène ou éthylène, avec un agent de clivage de l'éther.

7. Procédé suivant la revendication 6, dans lequel l'agent de clivage de l'éther est le tribromure de bore dans un solvant organique halogéné.

8. Composition pharmaceutique présentant une activité contre l'angine de poitrine qui comprend un composé suivant les revendications 1,2,3,4 ou 5 en association avec un véhicule pharmaceutique,

9. Composition pharmaceutique, sous forme d'unité posologique présentant une activité contre l'angine de poitrine, qui comprend un composé suivant l'une quelconque des revendications 1 à 5, en une quantité non toxique, suffisante pour produire une activité contre l'angine de poitrine chez un humain, en association avec un véhicule pharmaceutique.

10. Composition pharmaceutique suivant la revendication 9 comprenant de 15 à 75 mg d'un composé de l'une quelconque des revendications 1 à 5 par unité posologique.

**Revendications pour l'Etat contractant: AT**

1. Procédé de préparation d'un composé de formule:

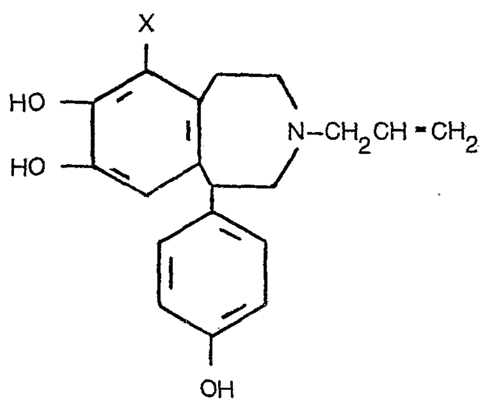

I

dans laquelle X représente un groupe halo; ainsi qu'un sel d'addition de ce composé avec des acides pharmaceutiquement acceptables ou son ester O-alcanoylé inférieur, chacun des groupes alcanoylés ayant de 2 à 8 atomes de carbone, qui consiste à faire réagir un composé de formule:

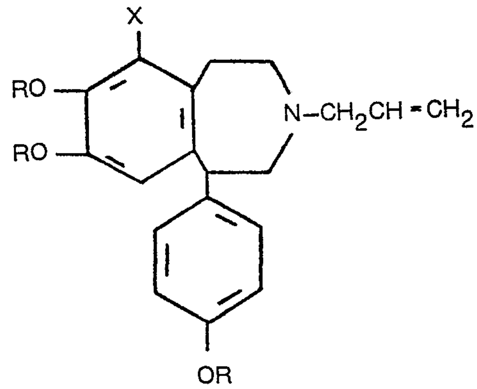

II

dans laquelle X représente un groupe halo et chacun des groupes R, qui sont identiques ou différents, représente un groupe alcoyle inférieur contenant de 1 à 6 atomes de carbone, un groupe benzyle, ou lorsqu'ils sont considérés conjointement aux positions 7, 8 du cycle benzénique, représentent un groupe méthylène ou éthylène, avec un agent de clivage de l'éther et à transformer éventuellement le produit en un sel d'addition d'acide pharmaceutiquement acceptable ou en ester O-alcanoylé inférieur.

2. Procédé suivant la revendication 1, dans lequel X représente un groupe chloro.

3. Procédé suivant la revendication 1, dans lequel X représente un groupe iodo.

4. Procédé suivant l'une quelconque des revendications 1 à 3, dans lequel tous les trois groups R représentent un groupe méthyle.

5. Procédé suivant l'une quelconque des revendications 1 à 4, dans lequel l'agent de clivage de l'éther est le tribromure de bore dans un solvant organique halogéné.